Europäisches Patentamt

(19) European Patent Office

Office européen des brevets

(11) Publication number: **0 265 253**
A2

# (12) EUROPEAN PATENT APPLICATION

(21) Application number: 87309324.9

(22) Date of filing: 21.10.87

(51) Int. Cl.⁴: **C 12 N 11/08**
C 12 N 11/04, C 12 N 11/12,
C 12 Q 1/60, C 12 P 1/00,
A 61 K 9/52, C 02 F 3/00

(30) Priority: 24.10.86 US 922517

(43) Date of publication of application:
27.04.88 Bulletin 88/17

(84) Designated Contracting States:
AT BE CH DE ES FR GB GR IT LI LU NL SE

(71) Applicant: **KINGSTON TECHNOLOGIES LIMITED PARTNERSHIP**
2235-B Route 130
Dayton New Jersey 08810 (US)

(72) Inventor: **Thakore, Yatin B.**
11 Rues Lane
East Brunswick New Jersey 08816 (US)

(74) Representative: **Seaborn, George Stephen et al**
c/o Edward Evans & Co. Chancery House 53-64 Chancery Lane
London WC2A 1SD (GB)

(54) **Stabilized dispersed enzyme.**

(57) Enzymes are immoblized, stabilized and evenly dispersed without destroying their activity by sequestering them within micropores of a solid carrier structure and filling the pores with a water-immiscible organic liquid, in which the enzyme remains active. The preferred solid carrier is a microporous membrane. The system is manufactued by providing a solid carrier having a microporous surface whose pores contain an aqueous solution, allowing an aqueous solution of enzyme to equilibrate with the solution in the pores, causing a controlled partial collapse of the pores to effectively trap the enzymes, and replacing the liquid in the pores with a water-immiscible organic liquid. The system can be used to assay for or produce an organic compound. It also can be used for slow release of an enzyme.

Membrane (containing water)
↓ equilibrate with aqueous enzyme

Membrane (containing aqueous enzyme)
↓ partially dry to effect controlled collapse of pores

Reduced aqueous content of Membrane and entrapping enzymes
↓ contact with water miscible solvent

Membrane (containing enzyme) in water-miscible organic liquid
↓ contact with water-immiscible organic liquid

Stabilized, dispersed enzyme in membrane

FIG.1.

**Description**

STABILIZED DISPERSED ENZYME

This invention relates to enzymatic processes, e.g., for assaying or synthesizing specific organic compounds.

Background of the Invention

Enzymatic reactions are widely used in various biomedical and industrial applications such as assaying or synthesizing specific organic compounds. To facilitate enzyme handling and recovery, enzymes have been immobilized on insoluble supports, including membranes. Techniques for immobilization include chemical cross-linking, covalent binding to supports, physical entrapment or adsorption, or a combination of physical and chemical processes.

To be functional, enzymes must retain their naturally occuring folding pattern which establishes the configuration or structure necessary for enzymatic actiyity. Denaturization of the enzyme (alteration of its folding pattern accompanied by loss of activity) can result from prolonged storage, heat, and other environmental factors. Many immobilization methods, particularly covalent binding and cross-linking, have been proposed to preserve the native conformation of the enzymes, but such methods themseles may contribute to enzyme inactivation.

Kazandjian et al., Biotech. and Bioeng. XXVIII:417-421 (1986) disclose a method of precipitating two enzymes, horseradish peroxidase and cholesterol oxidase, onto a glass powder. First they form an aqueous slurry of enzyme and powder, and then they dry the slurry to obtain "visibly dry (free-flowing) beads." They add the resulting beads to enzyme substrate (p-amisidine) dissolved in various solvents, and conclude that the reaction proceeds fastest in

"very hydrophobic, water immiscible solvents that evidently do not strip the essential water from the enzyme even if no exogenous water is added (on top of that brought in with $H_2O_2$) .... [Even] in toluene and other highly hydrophobic organic solvents, a certain amount of water present is required."

They attribute loss of enzymatic activity in less hydrophobic, more water-miscible solvents to stripping of critical water molecules from the enzyme.

Klibanov, Science 219: 722-726 (1983) discloses various strategies for enzyme stabilization, including attaching the enzyme to a support by multiple links to avoid unfolding, and encapsulating the enzyme in membranes that are impermeable for enzymes, but permeable for low molecular weight substrates and products. Entrapment in microcapsules is accomplished by "interfacial polymerization, liquid drying or phase separation." Entrapment in liposomes or in hollow fibers is also disclosed.

Zaks and Klibanov, Science, 224:1249-1251 (1984) and Zaks and Klibanov, Proc. Nat'l Acad. Sci. 82:3192-3196 (1985) disclose that porcine pancreatic lipase, yeast lipase, and mould lipase retain their activity in nearly anhydrous organic solvents. They further disclose that, while water is essential for maintenance of activity of these enzymes, it also participates in inactivation processes, particularly thermal inactivation. They further disclose that enzymes are more heat-stable in organic, water-immiscible solvents.

Summary of the Invention

It has now been discovered that enzymes can be stabilized and evenly dispersed without destroying their activity by sequestering the enzyme at least at the surface of a solid carrier structure having micropores and filling the pores with a water-immiscible organic liquid, inwhich the enzyme remains active. The preferred solid carrier is a microporous membrane.

In preferred embodiments, the organic liquid is characterized by a dipole moment less than 2.5 Debye units, a dielectric constant less than 20, and a boiling point at atmospheric pressure of at least about 40°C but preferably over about 75°C. The system is particularly useful for enzymes such as cholesterol oxidase, whose substrates are insoluble or sparingly soluble (less than about 10g/l) in water under physiological conditions. Preferably the liquid content of the carrier is at least 90 percent water-immiscible organic liquid.

A second aspect of the invention generally features a method of making a stabilized, evenly dispersed enzyme system, by providing a solid carrier having a microporous structure whose pores contain an aqueous solution, allowing an aqueous solution of enzyme to equilibrate with the solution in the pores, and replacing the liquid in the pores with a water-immiscible organic liquid. Preferably, after equilibration, the enzyme pores are subjected to controlled partial collapse. By partial collapse, I mean that the pores are not eliminated, but they are collapsed sufficiently to effectively trap the enzyme. The preferred method of effecting controlled partial collapse and replacement of the aqueous liquid is to dry the carrier surface to remove water, introduce a water miscible organic liquid into the pores, and then contact the carrier with the immiscible organic liquid, allowing replacement by diffusion. Preferably, the largest membrane pores initially have a diameter in the range of 10 Angstroms to 100 microns. After controlled collapse, the pore size is reduced to sequester the enzyme (or cell debris containing the enzyme) to on the order of between 5 Angstroms and 10 microns.

The invention offers improved enzyme stability, e.g., as manifested by shelf-life in a diagnostic kit; moreover it substantially improves heat tolerance of the enzyme, e.g. so that its catalytic reaction can be performed at temperatures well above ordinary physiological temperatures. Also, the invention provides an even dispersion of enzyme without formation of clums or precipitation, which limit surface area contact with the reaction

0 265 253

medium. The system is produced under mild conditions so as to preserve enzymatic activity. The system can also be used for enzymes having water soluble substrates, by contacting the enzyme-containing membrane with substrate dissolved in an aqueous phase. Moreover, the system can be used to react substances in aqueous media over a wide pH range, because the organic liquid insulates the membrane from the pH of the surrounding aqueous medium.

The invention also can be used to provide sustained controlled release of active enzyme over time, as described more fully below.

Other features and advantages of the invention will be apparent from the following description of the preferred embodiment and from the claims.

Description of the Preferred Embodiment

Fig. 1 is a flow diagram of the steps for manufacturing an enzyme system.
Fig. 2 is a graph demonstrating the system of example 1.
Fig. 3 is a graph demonstrating the system of example 2.
Fig. 4 is a graph demonstrating the system of example 5.

The Enzyme System

Enzymes used in the above-described system may be virtually any known enzyme, but preferred enzymes are those catalyzing reaction of a lipophilic substance, i.e., one that prefers non-polar, hydrophobic water-immiscible liquids over polar liquids such as water; i.e. the substance should be sparingly (if at all) soluble in water, but easily dissolved in the water-immiscible organic liquid chosen.

The organic liquid or solvent used could be any apolar organic solvent which is immiscible with water. In general, the solubility of water in these organic solvents should be less than 10% and preferably less than 1% by weight. The dipole moment of these solvents in general will be between 0 and 2.5 and preferable under 1 Debye unit. The dieletric constants of these solvents in general will be under 20 and preferably under 10. It will be preferable to have solvents with relatively high boiling points specifically for applications involving higher temperatures. Examples of suitable solvents in this class include toluene, benzene, xylene, hydrocarbons, oils, higher alcohols etc. The organic liquid can be readily selected for a given enzyme as described herein. The final organic liquid content of the membrane should be over 90% of the total liquid in the membranes and preferable over 98% of the total liquid.

The preferred carriers are membranes, although other forms of carrier could be used. Specifically, the membrane can be in any convenient form such as flat sheet, hollow fibers, tubes, sponges or even porous rods or fabricated forms from basic membrane structures. Membranes with larger pores in the range of 10 Angstroms - 100 micron and/or initial void volumes between 20-97% of wet volume are suitable for these procedures, with pore sizes in 10 Angstroms - 5 micron and/or initial void volumes between 50-90% being the most preferred range.

The two primary requirements of membranes for such a procedure are that: i) the initial pore sizes of the membranes are large enough to allow the enzymes of interest to enter the membrane matrix by diffusion (in the case of asymmetric membranes, at least the pores on the more open spongy side are large enough for the enzymes to diffuse into the membrane matrix); and ii) the water (or other non-solvent) in the pores is in its non-equilibrium state, such that the pores can be collapsed irreversibly upon drying. These requirements can be easily met by membranes synthesized from a wide variety of relatively hydrophobic polymers or their derivatives, as described below. Also the membrane should be resistant to the organic solvent of interest.

As noted, the initial water (non-solvent) content should be such that, upon drying or evaporation of the non-solvent, the pores will collapse irreversibly. This requirement is easily met by membranes fabricated from a wide variety of relatively hydrophobic polymers, copolymers or their derivatives such as nylons, polyesters, polysulfones, polyacrylonitriles, polycarbonates, polyvinylchlorides, cellulose esters etc. When solutions of these membranes are cast, spun or extruded and allowed to coagulate in a non-solvent bath or in atmoshperic humidity, the membranes contain a high fraction of non-solvent, but upon drying the pore-structure generally collapses irreversibly such that they will not regain the original amount of water or non-solvent upon rewetting.

Alternatively, the membrane could be hydrophilic such that the pores would collapse and the membranes would deswell when water or hydrophilic solvent is replaced by non-polar solvents. Such requirements are also easily met by a number of common polymers such as poly-HEMA or their derivatives, cellulosic or their derivatives, hydrolyzed polyacrylonitrile or their derivatives, collagen, polyvinyl alcohol or their derivatives, etc.

The choice of hydrophobic or hydrophilic polymer matrices depends upon the application. In the case of hydrophilic membranes, the entrapment as described above will be due to the desolvation and consequent partial collapse of the membrane structure in presence of organic liquids. When such membranes are used in contact with aqueous liquids, e.g., as in diagnostic strips, the membranes would gradually reswell and water will preferentially displace the entrapped organic liquids and cause some of the immobilized enzymes to leach into the analate solution. Such loss of enzymes would be of little consequence in the case of disposable diagnostic strips but is not desirable for applications requiring continuous processes as for examle in bioreactors.

For continuous processes conducted in contact with aqueous or hydrophilic media, hydrophobic membrane matrices which undergo permanent shrinkage of pores will be more desirable. The hydrophilic membrane matrices, however are preferred for controlled release of enzymes, because the enzymes would be

3

stable for a long time but could be released slowly into external aqueous media when contacted by such media. Such systems are useful for example in therapeutic type of application or for example in enzymatic processes involving in situ clean up of soil or water contamination.

Enzyme stabilization in hydrophilic matrices can also be used as a storage device to keep the enzymes active until needed. This would be particularly useful for in-field applications where it may not be practicable to have refrigeration. At the time of use, stabilized enzymes trapped in hydrophilic matrices can release the enzymes into outside aqueous solutions. Such stabilized enzymes therefore can also be used with applciations requiring hydrophilic substrates and/or cofactors, both of which are largely insoluble in the organic liquids.

Manufacture

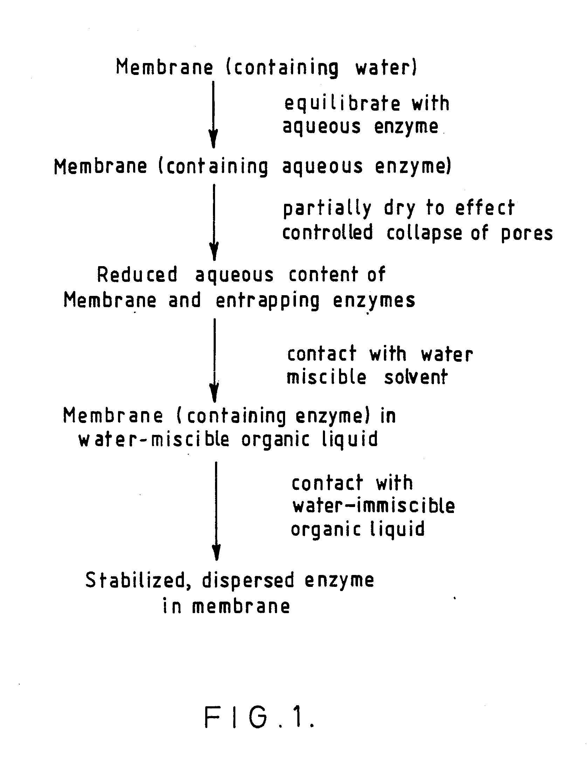

One convenient way to manufacture these stabilized membranes under mild conditions (shown in Fig. 1) is to start with a suitable synthetic membrane containing water or aqueous buffer solution in its pores and with pore-sizes large enough to accommodate the enzyme molecules. The membrane is next placed in a buffered solution of enzyme at its optimum pH and the enzyme is allowed to diffuse into the membrane matrix. After the diffusional exchange, the membrane is partially dried either by gently squeezing the membrane between paper towels or by subjecting it to vacuum. The membrane is of such a type that this partial drying causes partial collapse of the pores, thereby effectively entrapping the enzyme molecules within the pores.

After the entrapment step, the membrane is next placed in a water miscible organic solvent such as acetone, ethanol, etc., and the water in the membrane is exchanged via diffusional exchange. Once this exchange is complete, the enzyme-entrapped membrane is further placed into the desired organic liquid. The diffusional exchange causes the enzyme molecules to be surrounded by the appropriate organic liquids. Since the enzymes are effectively trapped within the pores or void spaces of the polymer matrix, they do remain well-dispersed during the diffusional exchange. The hydrophilicity of parts of the enzyme will result in a small amount of bound water enveloped around the enzyme molecules. Based on current theoretical understanding, it appears that the bound water around enzyme molecules ensures their normal functioning, and the hydrophobic medium surrounding them establishes hydrophobic interaction to prevent unfolding of the enzyme molecules.

Although the above method is a gentle and straightforward method for making such membranes, there are a variety of other common methods of immobilization which are familiar to those in the field, including cross-linking and entrapment, covalent binding and entrapment, etc. These methods generally could be used for the present invention, but the particular method chosen should be selected to avoid damage to the enzymes. The membrance pores or void spaces should be filled with appropriate organic solvent as soon as possible, subsequent to immobilization.

Finally, even though the present invention describes stabilization of enzymes which are isolated and not a part of living or dead cells, it is possible to prepare such membranes from cell fragments or even whole cells without isolating the enzymes from the cells. The use of the invention is not limited by whether the enzymes are in isolated and purified form or part of cell fragments and therefore in crude, unpurified forms.

The following examples illustrate the invention and they are not to be construed as limitations on it. In these examples, "DI" is the abbreviation for deionized and "TChA" stands for the sodium salt of tarrocholic acid.

Example 1:

Microporous membranes were made by dissolving polyacrylonitrile (molecular weight 150,000) in DMSO at a concentration of 6% by weight, and the solution was filtered through 5 micron stainless steel wire-mesh. The filtered solution was cast on a glass plate to a 10 mil (250 micron) thickness with a casting knife, and allowed to coagulate at 70°F and between 70-75% humidity. The polymer solution coagulated in 2-3 hours, giving a microporous membrane with maximum pore-size of 0.8 microns as judged by bubble point. The water content of this membrane was 94% by weight.

The membrane was washed with water for several days and punched into discs of 25 mm diameter. These discs were then equilibrated overnight with 0.1 M solution of phosphate buffer at pH 7 at 4°C in a refrigerator, and the next day they were equilibrated in a enzyme solution containing cholesterol esterase, cholesterol oxidase (microbial) and horseradish peroxidase at pH 7 in 0.1 M phosphate buffer at 4°C. The concentrations of the three enzymes in the buffer solution were 237.5 units/liter, 507.5 units/liter, and 72,250 units/liter respectively.

After allowing about 5 hours for the diffusional exchange, the enzyme-entrapped membrane discs were partially dried by placing them between Kimwipes® (Kimberly-Clark Corp.) in stacks of two, and lightly squeezing them for 5 minutes. During this time, the water content dropped from 94% to between 50 and 70%, and the pores shrank sufficiently to retain even molecules under 10,000 daltons molecular weight.

After this partial collapse of the pores, the films were placed in acetone for about 2 hours, during which time acetone was replaced with fresh acetone at least four times. After this acetone exchange, the enzyme-containing discs were divided into 3 different lots: One lot was placed in dodecane, one lot in 1-heptanol, and one lot in toluene. A few films after partial drying step were saved as controls without subjecting them to acetone or solvent exchange. The solvents also contained chromophores and activator in the following concentration: 4-aminoantipyrine at 4.9 m mol/liter; phenol 106.3 m mol/liter and sodium salt of taurocholic acid at 150 mg/liter. (These reagents were not fully soluble in dodecane.) The films were allowed to stay in the solvents for 2 days at 4°C during which time fresh solvent was exchanged.

After two days the enzyme containing films were removed from solvent, surface dried, and stored in the refrigerator at 4°C until further needed. These films were tested for their ability to detect total cholesterol.

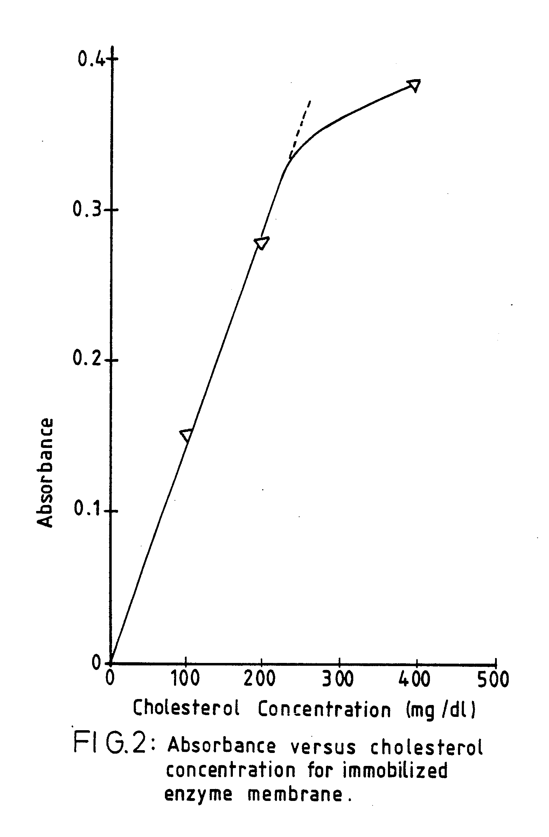

The response to cholesterol concentration was obtained in the following manner: To an enzyme film in a vial, 1 ml of 6% TChA solution in 0.1 M phosphate buffer and .04 ml of cholesterol standard solution (between 100 - 400 mg/dl cholesterol) were added and the vial was incubated at 37°C for an hour. The resultant solution in the vial was diluted fourfold with DI water and resultant pinkish color observed against a blank at 500 nm in a visible spectrophotometer. The blank had identical composition except, instead of cholesterol, de-ionized water was added. The response was proportional to cholesterol concentration, with tolune films showing the strongest color. The response of toluene film is shown in Figure 2.

Example 2:

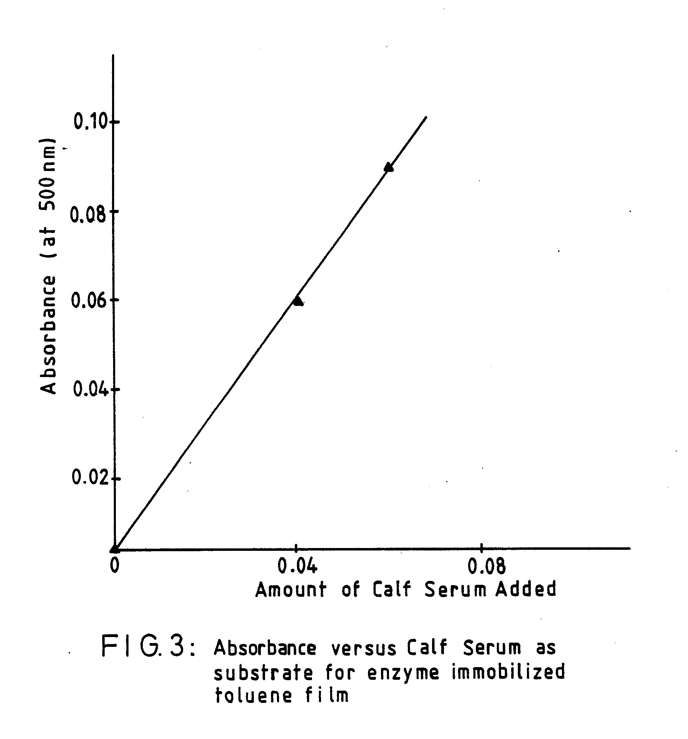

Two toluene films of Example 1 were taken in separate vials and to these 1 ml of 6 g% TChA solution in 0.1 M phosphate buffer was added followed by 0.04 and 0.08 ml of calf serum respectively. The vials were then incubated for 1 hour at 37°C and the solutions were diluted fourfold with DI water. The resultant pinkish colors were measured at 500 mm against a blank (treated identically but without the serum). The response is shown in Figure 3 is proportional to the amount of calf serum. The cholesterol in the calf serum was determined to be 55 mg/dl as judged from the results of Figure 2.

Example 3:

Ultrafiltration membranes were made by dissolving polyacrylonitrile in DMSO at a concentration of 6% by weight and the solution filtered through 5 micron stainless steel wire mesh. The filtered solutions were cast on glass plates with a doctor knife, with a casting thickness of 10 mils and immediately coagulated in DI water at room temperature. The resultant membranes were of ultrafiltration type, with a shiny skin and porous substructure. The molecular weight cut-off was about 100,000 but the pores on the underside were large enough to allow blue dextran (mol. wt. 2,000,000 daltons) to penetrate through.

The procedure for enzyme immobilization in this membranes was identical as described in Example 1 and the results were similar.

Example 4:

The films of examples 1 and 3 were heated in vials at 60°C for 3 hours. The films containing immobilized enzyme in the aqueous buffer as well as free buffered enzyme solutions were also heated as controls. The free enzyme solutions were heated for only 1 hour at 60°C.

After heating, the vials were allowed to cool to room temperature and 0.25 ml chromophore and 0.75 ml of 6% TChA activator solution in phosphate buffer were added to the films. In the case of free enzyme solutions, to 0.5 ml of enzyme solution, 0.25 ml each of the chromophore and activator solutions were added. To each of these solutions, 0.04 ml of 400 mg/dl cholesterol standard was added and the solution was incubated at 37°C for 45 minutes. As a blank, 0.25 ml chromophore and 0.75 ml of activator solution was heated in an identical manner followed by five fold dilution with DI water. The chromophore solution had 4.9 m mol/liter 4 aminoantiyrine and 106.3 m mol/liter of phenol in 0.1 M phosphate buffer.

The results are shown in Table 1 which clearly shows that the aqueous free enzymes as well as aqueous immobilized enzymes are virtually inactive whereas the organic solvent immobilized films retain their activities. Toluene films have the highest thermal stabilities.

Example 5:

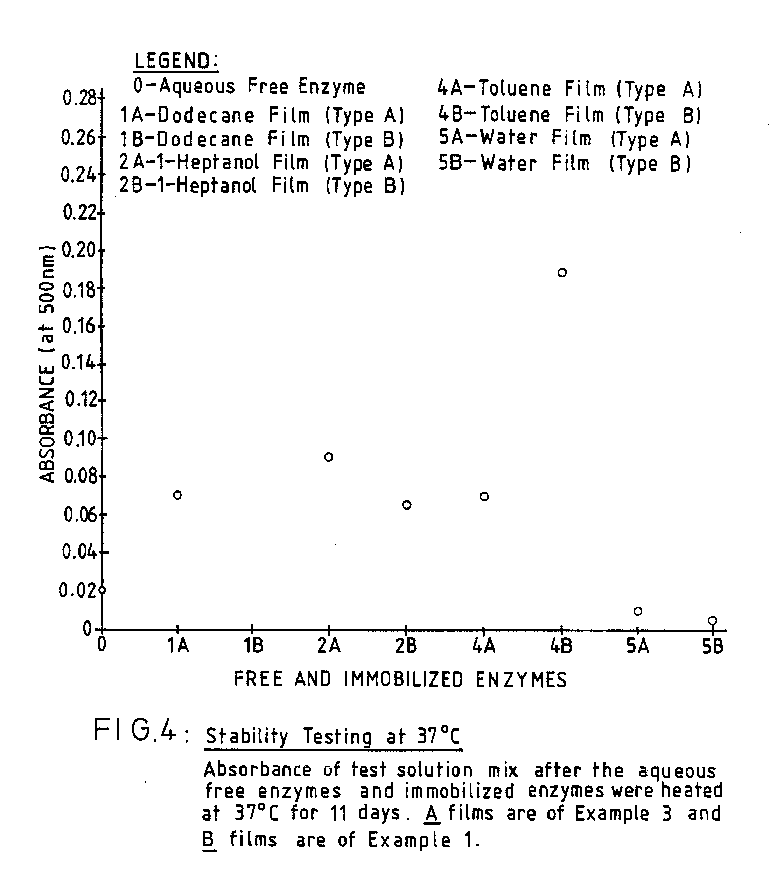

The solvent immobilized films along with aqueous buffered enzyme solution and aqueous enzyme immobilized films were incubated in an oven for 11 days at 37°C. The films and the control aqueous solution were analyzed as per example 3. The results are shown in Figure 4.

The trend is similar to that observed at 60°C. The aqueous enzyme solution is minimally active. Considering that the aqueous solution of enzymes (0.5 ml. volume) had approximately five times the enzymes as compared to immobilized enzyme discs (0.12 ml volume), the toluene film of Example 1 were at least 35 - 40 times as active as compared to free aqueous solution or aqueous immobilized films of Example 1. The toluene films of Example 3 were at least 15 times as active as compared to free native solutions. The other organic films with dodecane, and heptanol were also at least 15-20 times as active as the free enzyme solution.

TABLE 1: Effect of 60°C Heating on Enzyme Activity
Type A films are those of example 3 and Type B
films are those of example 1. Increasing color
intensity represents increasing activity.

| | |
|---|---|
| Aqueous enzyme solution and Type B aqueous film | No color |
| Type A aqueous film | Very faint pink |
| Type A 1-heptanol film and Type B 1-heptanol film | Light orange |
| Type B dodecane film | Light pink (turbid) |
| Type A dodecane film | Pink (clear) |
| Type A toluene film | Dark orangish pink |
| Type B toluene film | Very dark orangish pink |

(Increasing colour intensity

## Claims

1. A stable, evenly dispersed enzyme composition comprising:
   a) a solid carrier having a microporous structure; and
   b) an enzyme sequestered within the carrier pores at least at the carrier surface, the pores being filled with a water-immiscible organic liquid, the enzyme micro-environment comprising sufficient water to allow enzymatic activity in the organic liquid.

2. A composition as in claim 1, in which the solid carrier is a microporous membrane.

3. A composition as in either preceding claim, in which the pores are on the order of 5 Angstoms to 10 microns in diameter.

4. A composition as in any preceding claim, in which the enzyme substrate is insoluble or sparingly soluble in water.

5. A composition as in any preceding claim, in which the organic liquid has a dipole moment of less than 2.5 Debye units.

6. A composition as in any preceding claim, in which the organic liquid boiling point is at least 40°C.

7. A composition as in any preceding claim, in which the organic liquid has a dielectric constant less that 20.

8. A composition as in any preceding claim, in which at least 90% of the liquid content of the carrier is

**0 265 253**

water immiscible organic liquid.

9. A composition as in claim 2 or any of claims 2 to 8 as appendant to claim 2, in which the membrane comprises polyacrylonitrile.

10. A method of making a stabilized, evenly dispersed enzyme system comprising:
providing a solid carrier having a microporous structure, the pores being filled with an aqueous liquid;
allowing an aqueous enzyme solution to equilibrate with the aqueous liquid in the pores;
replacing aqueous liquid in the pores with a water-immiscible organic liquid.

11. The method of claim 10, wherein, after the enzyme solution is equilibrated, the method comprises causing a controlled, partial collapse of the pores to effectively sequester the enzyme.

12. The method of claim 11, the controlled collapse and aqueous liquid replacement comprises removing aqueous liquid from the pores, introducing a water-miscible organic liquid into the pores, and contacting the carrier with the water-immiscible organic liquid to replace the water-miscible organic liquid by diffusional exchange.

13. A method of determining the presence of a substance, comprising:
providing the system of any of claims 1 to 9, wherein the enzyme catalyzes a reaction of the substance being assayed to produce a product;
contacting the system with a solution containing the substance being assayed; and
determining the presence of the reaction product.

14. The method of claim 13, wherein the substance is cholesterol or cholesterol esters, and the enzymes are cholesterol oxidase, cholesterol esterase and peroxidase.

15. A method of enzymatic production of a product from a substrate compound, comprising
providing the system of any of claims 1 to 9, wherein the enzyme catalyzes conversion of the substrate compound into the product;
contacting a solution containing the substrate compound with the system; and
recovering the product.

16. A method of sustained controlled release of an enzyme to an aqueous medium, comprising
providing the system of any of claims 1 to 9, wherein the enzyme is larger than the carrier pores, and the carrier is an aqueous fluid swellable polymer, and
exposing the system to the aqueous medium, wherein, the aqueous medium enters the pores, causing them to swell to release the enzyme.

7

Membrane (containing water)

        equilibrate with
        aqueous enzyme

Membrane (containing aqueous enzyme)

        partially dry to effect
        controlled collapse of pores

Reduced aqueous content of
Membrane and entrapping enzymes

        contact with water
        miscible solvent

Membrane (containing enzyme) in
water-miscible organic liquid

        contact with
        water—immiscible
        organic liquid

Stabilized, dispersed enzyme
in membrane

# FIG.1.

FIG.2: Absorbance versus cholesterol
concentration for immobilized
enzyme membrane.

0265253

16·11·07

FIG.3: Absorbance versus Calf Serum as substrate for enzyme immobilized toluene film

LEGEND:
0-Aqueous Free Enzyme
1A-Dodecane Film (Type A)
1B-Dodecane Film (Type B)
2A-1-Heptanol Film (Type A)
2B-1-Heptanol Film (Type B)
4A-Toluene Film (Type A)
4B-Toluene Film (Type B)
5A-Water Film (Type A)
5B-Water Film (Type B)

FIG.4: Stability Testing at 37°C

Absorbance of test solution mix after the aqueous free enzymes and immobilized enzymes were heated at 37°C for 11 days. A films are of Example 3 and B films are of Example 1.